# FASCICULE DE BREVET EUROPEEN

(11) **EP 0 730 850 B1**
(45) Date de publication et mention de la délivrance du brevet: **13.09.2000**
(21) Numéro de dépôt: 96400384.2
(22) Date de dépôt: 23.02.1996
(51) Int. Cl.: A61F 5/01

(54) **Halo réglable fixable sur le crâne d'un patient**
Verstellbarer an den Schädel eines Patienten ansetzbarer Kopfring
Adjustable halo attachable to a patient's skull

(30) Priorité: 09.03.1995 FR 9502758
(43) Date de publication de la demande: 11.09.1996
(73) Titulaire: ETABLISSEMENTS PROTEOR Société anonyme dite:, 21100 Dijon (FR)
(72) Inventeur: Guigui, Pierre, Dr., 75016 Paris (FR); Mazda, Kervan, Dr., 75012 Paris (FR); Pierron, Olivier, 1220 Luxembourg (LU)
(74) Mandataire: Jolly, Jean-Pierre

(56) Documents cités:
- WO-A-88/04541
- US-A- 5 156 588

## Description

La présente invention concerne un halo constitué d'éléments réglables en position et apte à être adapté en une pluralité de positions réglables sur le crâne d'un patient.

On sait que, dans le traitement de diverses affections de la colonne vertébrale, dues à des blessures ou des déformations, il est nécessaire d'exercer une traction sur la colonne vertébrale, au niveau des vertèbres cervicales. Dans ce but, on fixe sur la boîte crânienne du patient, à l'aide de vis pointeaux ou similaires, une structure rigide, qui a souvent un profil circulaire, d'où elle a tiré son nom de "halo", et ce halo est rendu rigidement solidaire, dans une position déterminée, d'une ossature solidaire de la cage thoracique du patient, par l'intermédiaire d'un corset par exemple.

L'invention vise à proposer un halo qui soit à la fois réglable en dimensions et réglable en position sur le crâne du patient, de manière à pouvoir être adapté à des morphologies et des dimensions de boîtes crâniennes différentes et à réduire ainsi le nombre de halos en attente d'utilisation.

A cet effet, l'invention a pour objet un halo comportant au moins deux barres fronto-temporales, assemblées entre elles et destinées à enserrer le front et les tempes du patient, et deux demi-arceaux, assemblées entre eux et solidaires des barres fronto-temporales, les demi-arceaux assemblés étant destinés à former un arceau enserrant la zone postéro-supérieure du crâne du patient. Ce type de halo fait partie de l'art de la technique antérieure interne. L'invention est caractérisée en ce que les barres fronto-temporales et/ou les demi-arceaux sont assemblés à l'aide de barrettes recouvrant leurs extrémités contiguës, les parties en regard des barres fronto-temporales, des demi-arceaux et des barrettes comprenant des orifices permettant de les rendre solidaires à l'aide de moyens d'assemblage tels que des vis, certains au moins des orifices des barrettes et/ou des barres fronto-temporales et/ou des demi-arceaux, se présentant sous la forme de lumières oblongues, permettant de régler le recouvrement des barrettes et des extrémités des pièces associées et d'adapter ainsi les dimensions du halo à la morphologie du crâne du patient.

Les barres fronto-temporales et les demi-arceaux pourront être assemblés directement entre eux et, dans ce cas, ces pièces comporteront des parties d'extrémité contiguës à recouvrement mutuel, dans lesquelles seront pratiqués des orifices permettant de les rendre mutuellement solidaires à l'aide de moyens d'assemblage tels que des vis, certains des orifices des parties d'extrémité associées des barres fronto-temporales et/ou des demi-arceaux se présentant sous la forme de lumières oblongues permettant de régler leur recouvrement et d'adapter ainsi les dimensions du halo.

Les demi-arceaux pourront également être réunis aux barres fronto-temporales par des pièces de jonction recouvrant partiellement les extrémités contiguës des barres fronto-temporales et, dans de cas, des orifices seront pratiqués dans les parties en regard de ces pièces de jonction et des barres fronto-temporales, en vue de les rendre rigidement solidaires à l'aide de moyens d'assemblage tels que des vis, certains des orifices des pièces de jonction et/ou des barres fronto-temporales ayant la forme de lumières oblongues, afin de pouvoir les assembler en une pluralité de positions.

Dans cette forme de réalisation, les demi-arceaux et les pièces de jonction seront avantageusement réunis par des barrettes recouvrant leurs extrémités contiguës, des orifices étant ménagés pour des moyens d'assemblage tels que des vis dans les parties disposées en regard des demi-arceaux et des pièces de jonction, certains des orifices de ces barres et/ou de ces pièces ayant la forme de lumières oblongues, de manière à pouvoir les assembler en une pluralité de positions.

Le halo comprendra également des barres occipitales, destinées à venir s'appliquer en regard de l'occiput du patient. Ces barres occipitales seront solidaires des extrémités des demi-arceaux ou des pièces de jonction contiguës aux barres fronto-temporales et, en vue de pouvoir les assembler en une pluralité de positions à l'aide de moyens d'assemblage tels que des vis, pour pouvoir choisir la position qui est la plus adaptée à la morphologie du crâne du patient, on prévoira, dans les extrémités des demi-arceaux, des pièces de jonction et/ou des barres occipitales, des orifices disposés en regard, dont certains au moins auront la forme de lumières oblongues.

L'invention apporte donc un moyen simple et facile à mettre en oeuvre pour adapter les dimensions du halo à celles du crâne du patient à équiper de ce halo et à sa morphologie.

De préférence, les vis pointeaux ou similaires utilisées pour fixer le halo sur la boîte crânienne du patient seront engagées dans des lumières oblongues des barres fronto-temporales et des barres occipitales, de manière à pouvoir régler leur position, et leur diamètre sera légèrement inférieur à la largeur des lumières oblongues, de manière à pouvoir les orienter au mieux par rapport au crâne du patient.

Comme on le verra plus en détail ci-après, en vue de pouvoir imprimer simplement à l'ensemble crâne-rachis cervical un mouvement de flexion ou d'extension, l'extrémité d'un demi-arceau et/ou d'une barre fronto-temporale pourra avantageusement être montée pivotante par rapport à une première pièce, solidaire elle-même d'une seconde pièce réglable en position sur une tige verticale d'un support thoracique, ladite extrémité étant solidaire en déplacement d'un moyen porté par la première pièce et apte à être sollicité en translation par rapport à cette première pièce pr une vis ou similaire.

D'autres caractéristiques et avantages de l'invention ressortiront de la description détaillée qui va suivre, dans laquelle on se référera aux dessins annexés. Sur ces dessins :
La figure 1 est une vue éclatée d'une première forme de réalisation du halo conforme à l'invention ;
La figure 2 est une vue en perspective du halo de la figure 1, après assemblage des pièces constitutives ;
La figure 3 est une vue de détail à plus grande échelle de ce halo ;
La figure 4 est une vue analogue à la figure 1 d'une seconde forme de réalisation du halo conforme à l'invention ;
La figure 5 est une vue en perspective éclatée illustrant un mode de fixation du halo des figures 1 à 3 sur des tiges verticales d'un support thoracique.

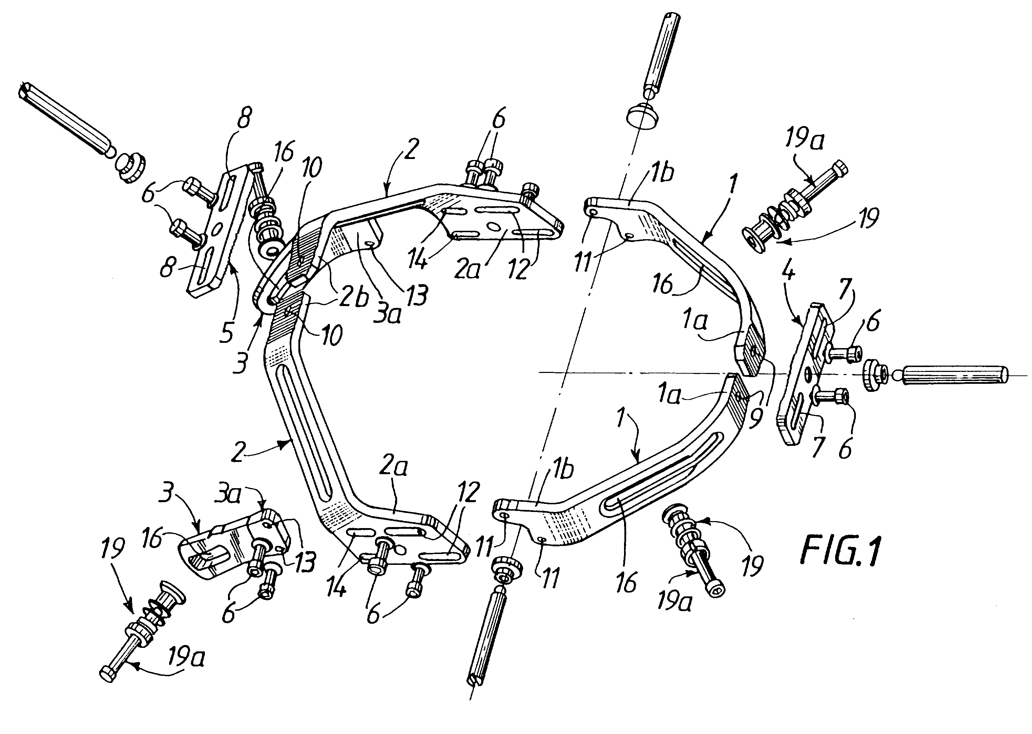

On se référera d'abord aux figures 1 à 3.

Le halo représenté sur ces figures comprend :
- deux barres fronto-temporales 1, destinées à venir s'appliquer respectivement en regard des zones fronto-temporales gauche et droite du crâne du patient et en regard de la zone frontale et à être assemblées entre elles ;
- deux demi-arceaux 2, destinés à être rendus solidaires des barres fronto-temporales et à être assemblés entre eux pour former un arceau qui coiffe la partie postéro-supérieure du crâne du patient ;
- deux barres occipitales 3, destinées à venir s'appliquer respectivement à gauche et à droite en regard des occiputs du patient et à être rendues solidaires d'une extrémité 2a recourbée des demi-arceaux 2 contiguë aux barres fronto-temporales associées.

L'invention vise à pouvoir adapter la forme et les dimensions d'un halo de ce type à des boîtes crâniennes de morphologies différentes.

Dans ce but, comme on le voit sur les dessins, les extrémités frontales contiguës 1a des barres 1 et les extrémités postéro-supérieures contiguës 2b des demi-arceaux 2 sont assemblées à l'aide de barrettes, respectivement 4 et 5, appliquées contre ces extrémités, et de vis 6 engagées, d'une part, dans des lumières oblongues, respectivement 7 et 8, des barrettes 4 et 5, et, d'autre part, dans des orifices circulaires, respectivement 9 et 10, pratiqués dans les extrémités 1a et 2b en regard des lumières oblongues 7 et 8.

Il est ainsi possible d'assembler entre eux, d'une part, les barres fronto-temporales et, d'autre part, les demi-arceaux 2, en une pluralité de positions, en décalant respectivement les extrémités contiguës 1a ou 2b pour les adapter à la forme de la boîte crânienne du patient.

De façon analogue, l'extrémité 1b des barres fronto-temporales opposée à l'extrémité 1a de celles-ci vient s'appliquer contre l'extrémité contiguë 2a des demi-arceaux 2 pour amener des orifices 11 de l'extrémité 1b dans les axes des deux lumières oblongues 12 parallèles ménagées respectivement dans l'extrémité 2a contiguë des demi-arceaux 2. Il est ainsi possible de décaler longitudinalement l'une par rapport à l'autre les extrémités 1b et 2a contiguës pour choisir la position d'assemblage la mieux adaptée à la morphologie du crâne du patient.

Enfin, selon le même principe, une extrémité 3a des barres occipitales vient s'appliquer contre une face de l'extrémité 2a contiguë du ou des demi-arceaux 2 associés, de manière à amener deux orifices 13 de l'extrémité 3a dans l'axe des deux lumières oblongues 14 des extrémités 2a. De cette manière, il est donc ici encore possible d'assembler à l'aide de vis 6, en une pluralité de positions, après translation et/ou rotation, la barre occipitale 3 et le demi-arceau 2 associé, pour s'adapter à la forme de la boîte crânienne du patient.

Il est naturellement également souhaitable de pouvoir adapter à la morphologie du crâne du patient la position et l'orientation des vis pointeaux qui servent à fixer le halo sur la boîte crânienne.

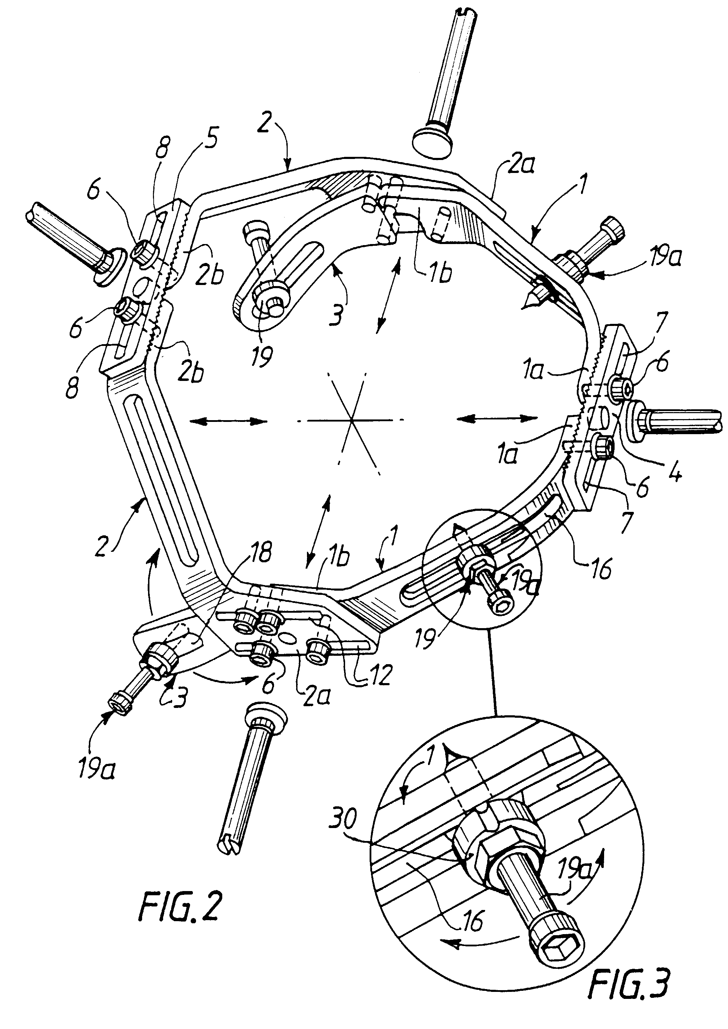

Dans ce but, des lumières oblongues 16 sont ménagées respectivement dans les barres fronto-temporales 1 et dans les barres occipitales 3, pour recevoir les guides-supports 19 des vis pointeaux 19a, qui sont maintenues en position par des rondelles et des écrous. Afin que les guides-supports 19 puissent être orientés de la manière la plus judicieuse, ils ont une portée légèrement sphérique, un méplat sur le corps cylindrique évitant la rotation dans les lumières oblongues, ce qui permet de les faire pivoter de quelques degrés, comme on le voit sur la figure 3, avant de les verrouiller en position à l'aide d'écrous 30.

On notera que les parties des barrettes 4 et 5 en regard des parties 1a des barres 1 ou des parties 2b des demi-arceaux 2 ont des surfaces crantées de profil complémentaire qui préviennent tout risque de glissement relatif accidentel des surfaces en contact. On pourrait substituer aux surfaces crantées tout autre moyen d'immobilisation connu dans la technique.

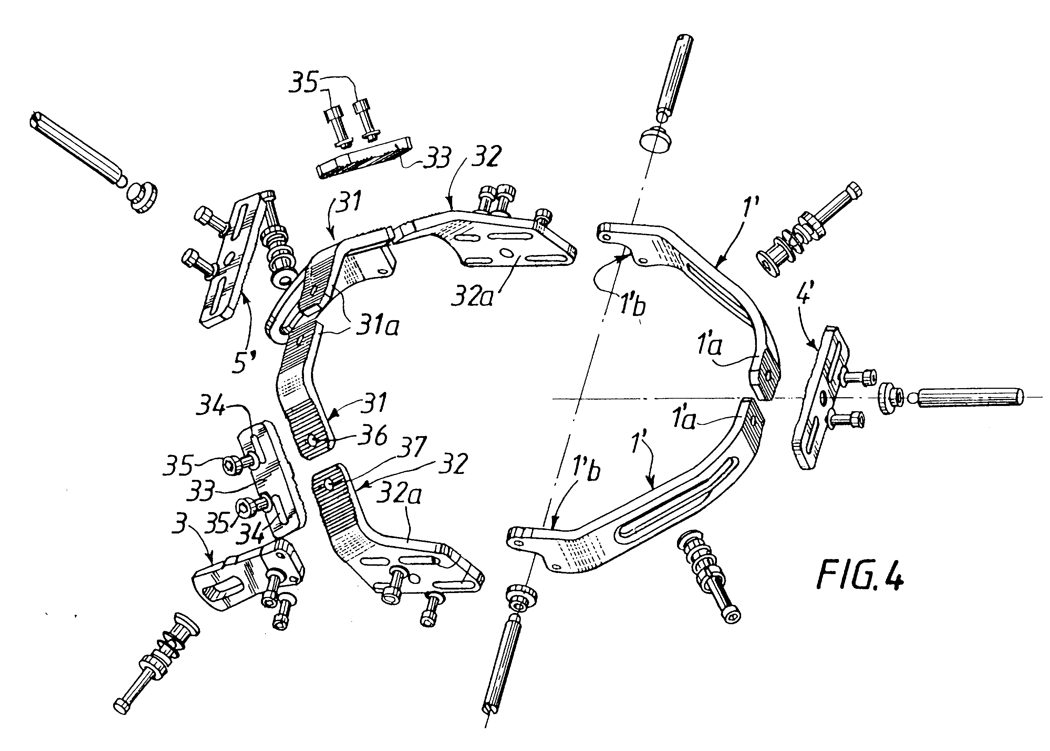

Dans la variante de la figure 4, on retrouve pour l'essentiel, les mêmes organes constitutifs que dans la forme de réalisation de la figure 1. Les organes déjà décrits sont désignés par les mêmes chiffres de référence affectés de l'indice ' et ils ne seront pas décrits à nouveau. La seule différence avec la figure 1 est que les demi-arceaux 2 sont remplacés chacun par deux parties distinctes assemblables en une pluralité de positions réglables, à savoir :
- une partie 31, à laquelle est attenante une extrémité recourbée 31a, qui coopère avec la barrette 5' ;
- une partie 32, à laquelle est attenante une extrémité recourbée 32a, qui coopère avec l'extrémité 1'b de la barre fronto-temporale 1'.

Une barrette 33 percée de deux lumières oblongues 34, permettant d'assembler les parties 31 et 32 en une pluralité de positions réglables, à l'aide de vis 35, que l'on engage dans la position désirée dans les lumières 34 et dans des orifices 36 et 37 percés respectivement dans les parties 31 et 32.

Dans cette forme de réalisation, il est ainsi possible de régler la hauteur des demi-arceaux constitués par l'assemblage des pièces 31 et 32, pour les adapter à la morphologie du patient.

Le halo conforme à l'invention peut être rendu solidaire de toute ossature de support thoracique utilisée dans la technique.

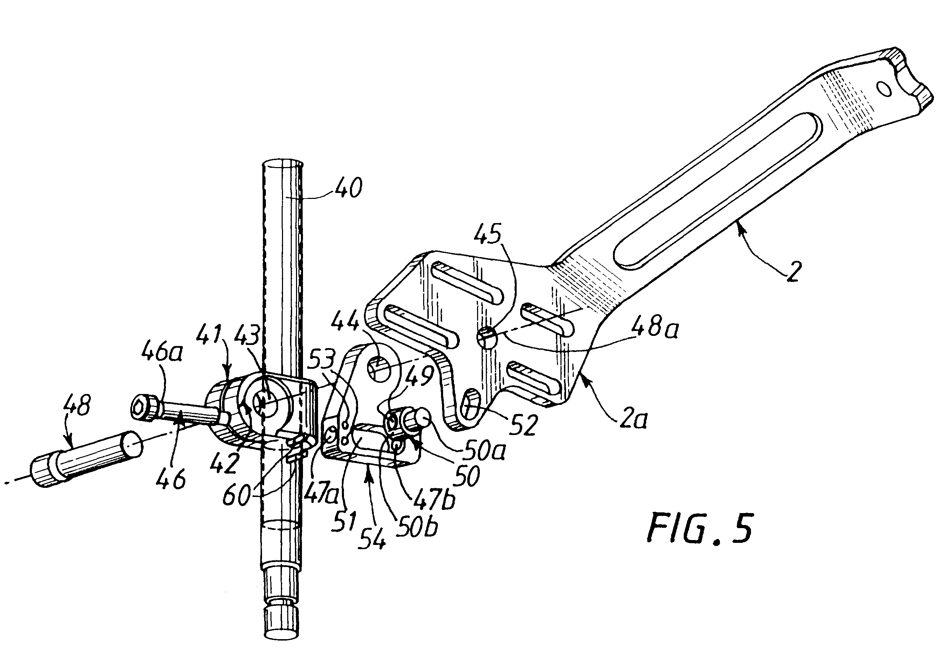

La figure 5 est une vue en perspective éclatée illustrant un mode de fixation du halo de la figure 1 sur deux tiges cylindriques verticales 40 d'un tel support thoracique.

Une pince 41 en forme de U est vissée sur chaque tige filetée 40 et comporte un lamage 42 dans lequel est logée et immobilisée en rotation dans une partie de forme complémentaire d'une pièce auxiliaire 54, interposée entre la pince 41 et l'extrémité 2a du demi-arceau 2 associé. Une vis 48, engagée dans des orifices, respectivement 43, 44, 45, de la pince 42, de la pièce 54 et de l'extrémité 2a du demi-arceau 2, permet de solidariser ces organes, et son axe 48a sert d'axe de pivotement pour le demi-arceau 2.

Une vis 46 est engagée dans des trous lisses de guidage 47a, 47b, de la pièce 54 et vissée dans un trou fileté 49 d'un téton 50, dont une partie 50b est logée dans un trou oblong 51 de la pièce 54, dirigé suivant l'axe de la vis 46, tandis qu'une partie 50a fait saillie latéralement par rapport à la pièce 54 et est engagée dans un trou oblong 52 de la partie 2a, dans lequel elle peut coulisser. Deux goupilles 60 sont engagées dans des orifices 53 de la pièce 54, d'axe perpendiculaire à l'axe de la vis 46, et viennent se loger dans une gorge 46a de cette vis, ménagée au-dessous de la tête de celle-ci, afin de l'immobiliser en translation tout en lui laissant une liberté de mouvement en rotation.

Lorsque l'on entraîne la vis 46 en rotation, celle-ci imprime un mouvement de translation à la pièce 50 dans le trou oblong 51. La partie 50a de la pièce 50 se déplace simultanément dans le trou oblong 52 de la partie 2a, en imprimant ainsi au demi-arceau un mouvement de rotation autour de l'axe 48a, qui se traduit pour le halo, et par conséquent pour l'ensemble crâne-rachis cervical, par un mouvement de flexion ou d'extension, suivant le sens de la rotation.

## Revendications

1. Halo comportant au moins deux barres fronto-temporales (1), assemblées entre elles et destinées à enserrer le front et les tempes du patient, et deux demi-arceaux (2), assemblés entre eux et solidaires des barres fronto-temporales, les demi-arceaux assemblés étant destinés à former un arceau enserrant la zone postéro-supérieure du crâne du patient, ce halo étant caractérisé en ce que les barres fronto-temporales (1) sont assemblés à l'aide de barrettes (4) et/ou les demi-arceaux (2) sont assemblés à l'aide de barrettes (5) recouvrant leurs extrémités contiguës, les parties en regard des barres fronto-temporales, des demi-arceaux et des barrettes comprenant des orifices (7, 8, 9, 10, 11, 12) permettant de les rendre solidaires à l'aide de moyens d'assemblage tels que des vis (6), certains au moins des orifices des barrettes (1) et/ou des barres fronto-temporales et/ou des demi-arceaux, se présentant sous la forme de lumières oblongues (7, 8, 12), permettant de régler le recouvrement des barrettes et des extrémités des pièces associées et d'adapter ainsi les dimensions du halo à la morphologie du crâne du patient.

2. Halo selon la revendication 1, caractérisé en ce que les barres fronto-temporales (1) et les demi-arceaux (2) sont assemblés directement entre eux, ces pièces comportant des parties d'extrémité contiguës (1b, 2a) à recouvrement mutuel, dans lesquelles sont pratiqués des orifices (11, 12) permettant de les rendre mutuellement solidaires à l'aide de moyens d'assemblage tels que des vis (6), certains des orifices des parties d'extrémité associées des barres fronto-temporales et/ou des demi-arceaux se présentant sous la forme de lumières oblongues (12) permettant de régler leur recouvrement et d'adapter ainsi les dimensions du halo.

3. Halo selon la revendication 1, caractérisé en ce que les barres fronto-temporales (1) et les demi-arceaux (2) sont réunis par des pièces de jonction qui recouvrent partiellement les extrémités des barres fronto-temporales, des orifices étant pratiqués dans les parties en regard de ces pièces de jonction et des barres fronto-temporales, en vue de les rendre rigidement solidaires à l'aide de moyens d'assemblage tels que des vis, certains des orifices des pièces de jonction et/ou des barres fronto-temporales ayant la forme de lumières oblongues, afin de pouvoir les assembler en une pluralité de positions.

4. Halo selon la revendication 3, caractérisé en ce que les demi-arceaux (2) et les barres fronto-temporales (1) sont réunis par des barrettes recouvrant leurs extrémités contiguës, des orifices étant ménagés pour des moyens d'assemblage tels que des vis dans les parties disposées en regard des demi-arceaux et des pièces de jonction, certains des orifices de ces barres et/ou de ces pièces ayant la forme de lumières oblongues, de manière à pouvoir les assembler en une pluralité de positions.

5. Halo selon l'une des revendications 1 à 4, comprenant en outre des barres occipitales (3) destinées à venir à s'appliquer en regard de l'occiput du patient, caractérisé en ce que les barres occipitales (3) sont solidaires des extrémités (2a) des demi-arceaux (2) ou des pièces de jonction contiguës aux barres fronto-temporales, à l'aide de vis (6) ou similaires engagées dans des orifices (13, 14) des pièces à assembler, certains au moins de ces orifices ayant la forme de lumières oblongues (14).

6. Halo selon l'une des revendications 1 à 5, caractérisé en ce que les barres fronto-temporales (1) et/ou les barres occipitales (3) comprennent des lumières oblongues (16) à travers lesquelles sont engagées des guides-supports (19) de vis pointeaux (19a) ou similaires destinées à assurer la fixation du halo sur la boîte crânienne du patient.

7. Halo selon la revendication 6, caractérisé en ce que la portée des guides-supports (19) des vis pointeaux (19a) ou similaires est légèrement sphérique afin de pouvoir orienter les vis (19a).

8. Halo selon l'une des revendications 1 à 7, caractérisé en ce que les demi-arceaux sont constitués de deux pièces (31, 32) assemblables en des positions réglables.

9. Halo selon la revendication 8, caractérisé en ce que les parties (31, 32) des demi-arceaux sont assemblées à l'aide de barrettes (33) comportant des évidements oblongs (34) dans lesquels sont engagées en position réglables des vis (35) ou similaires, engagées également dans des orifices (36, 37) des parties (31, 32).

10. Halo selon l'une des revendications 1 à 9, caractérisé en ce que l'extrémité (2a) de l'un des demi-arceaux (2) et/ou l'extrémité (1b) d'une barre fronto-temporale (1) est montée pivotante par rapport à une première pièce (54), solidaire elle-même d'une seconde pièce (41), réglable en position sur une tige (40) d'un support thoracique, ladite extrémité étant solidaire en déplacement d'un moyen (50) porté par la première pièce (54) et apte à être sollicité en translation par rapport à cette première pièce (54) par une vis (46) ou similaire.

## Claims

1. Halo, comprising at least two fronto-temporal bars (1), which are fitted together and are intended to grip the forehead and the temples of the patient, and two half-cradles (2), which are fitted together and are integral with the fronto-temporal bars, the fitted half-cradles being intended to form a cradle which grips the upper posterior area of the cranium of the patient, this halo being characterised in that the fronto-temporal bars (1) are fitted by means of small bars (4) and/or the half-cradles (2) are fitted by means of small bars (5), which overlap their contiguous ends, the portions facing the fronto-temporal bars, the half-cradles and the small bars comprising openings (7, 8, 9, 10, 11, 12), which permit them to be made integral with the aid of assembly means, such as screws (6), at least some of the openings in the small bars (4,5) and/or in the fronto-temporal bars and/or in the half-cradles being in the form of oblong slots (7, 8, 12), which permit the overlapping of the small bars and the ends of the associated parts to be adjusted and the dimensions of the halo to be thus adapted to the morphology of the cranium of the patient.

2. Halo according to claim 1, characterised in that the fronto-temporal bars (1) and the half-cradles (2) are fitted directly together, these parts comprising contiguous end portions (1b, 2a) for mutual overlapping, in which end portions are provided openings (11, 12), which permit them to be made mutually integral with the aid of assembly means, such as screws (6), some of the openings in the associated end portions of the fronto-temporal bars and/or in the half-cradles being in the form of oblong slots (12), which permit their overlapping to be adjusted and the dimensions of the halo to be thus adapted.

3. Halo according to claim 1, characterised in that the fronto-temporal bars (1) and the half-cradles (2) are joined by connection parts which partially overlap the ends of the fronto-temporal bars, openings being provided in the portions facing these connection parts and in the fronto-temporal bars so as to make them rigidly integral with the aid of assembly means, such as screws, some of the openings in the connection parts and/or in the fronto-temporal bars having the configuration of oblong slots, so as to permit them to be fitted in a plurality of positions.

4. Halo according to claim 3, characterised in that the half-cradles (2) and the fronto-temporal bars (1) are joined by small bars which overlap their contiguous ends, openings being provided for assembly means, such as screws, in the portions which are disposed so as to face the half-cradles and the connection parts, some of the openings in these bars and/or in these parts having the configuration of oblong slots, so as to permit them to be fitted in a plurality of positions.

5. Halo according to one of claims 1 to 4, also comprising occipital bars (3) which are intended to be applied so as to face the occiput of the patient, characterised in that the occipital bars (3) are integral with the ends (2a) of the half-cradles (2), or with the connection parts which are contiguous with the fronto-temporal bars, by means of screws (6) or the like which are engaged in openings (13, 14) in the parts to be fitted, at least some of these openings having the configuration of oblong slots (14).

6. Halo according to one of claims 1 to 5, characterised in that the fronto-temporal bars (1) and/or the occipital bars (3) comprise oblong slots (16), through which are engaged supporting guide means (19) of punch-like screws (19a) or the like which are intended to ensure the securement of the halo on the braincase of the patient.

7. Halo according to claim 6, characterised in that the bearing of the supporting guide means (19) of the punch-like screws (19a) or the like is slightly spherical so as to permit the screws (19a) to be orientated.

8. Halo according to one of claims 1 to 7, characterised in that the half-cradles comprise two parts (31, 32) which can be fitted in adjustable positions.

9. Halo according to claim 8, characterised in that the portions (31, 32) of the half-cradles are fitted by means of small bars (33) comprising oblong slots (34), in which screws (35) or the like are adjustably engaged in position, said screws also being engaged in openings (36, 37) of the portions (31, 32).

10. Halo according to one of claims 1 to 9, characterised in that the end (2a) of one of the half-cradles (2) and/or the end (1b) of a fronto-temporal bar (1) is mounted so as to pivot relative to a first part (54), which is itself integral with a second part (41) and is adjustable in position on a rod (40) of a thoracic support, said end being integral in displacement with a means (50) carried by the first part (54) and capable of being transposed relative to this first part (54) by a screw (46) or the like.

## Patentansprüche

1. Extensor-Ring (Halo) mit wenigstens zwei frontotemporalen Stangen bzw. Schienen (1), die mit einander verbunden sind und zum Umschließen der Stirn und der Schläfen des Patienten bestimmt sind, sowie mit zwei Halbbögen (2), die miteinander und mit den Frontotemporal-Stangen bzw. -Schienen verbunden sind, wobei die Halbbögen im montierten Zustand zur Bildung eines die postero-superiore Zone des Patientenschädels umschließenden Bogens bestimmt sind,
**dadurch gekennzeichnet,** daß
die Frontotemporal-Stangen bzw. -Schienen mit Hilfe von Platten (4) montiert sind und/oder daß die Halbbögen (2) mit Hilfe von ihre angrenzenden Enden übergreifenden bzw. überlappenden Platten (5) montiert sind, daß die gegenüberstehenden Teile der frontotemporalen Stangen bzw. Schienen, der Halbbögen und der Platten Öffnungen (7, 8, 9, 10, 11, 12) aufweisen, welche deren Verbindung mit Hilfe von Montagemitteln wie beispielsweise schrauben (6) gestatten, wobei bestimmte von diesen Öffnungen der Platten (1) und/oder der Frontotemparalstangen bzw. -schienen und/oder der Halbbögen in Form von Langlöchern (7, 8, 12) vorliegen, welche eine Einstellung der Überlappung bzw. Überdeckung der Platten und der Enden der zugeordneten Teile und damit eine Anpassung der Abmessungen des Extensor-Rings (Halos) an die Morphologie des Patientenschädels gestatten.

2. Extensor-Ring (Halo) nach Anspruch 1, dadurch gekennzeichnet, daß die frontotemporalen Stangen bzw. Schienen (1) und die Halbbögen (2) direkt miteinander montiert bzw. verbunden sind, daß diese Stücke Teile aneinander grenzender Enden (1b, 2b) mit gegenseitiger Überlappung aufweisen, in welchen Öffnungen (11, 12) vorgesehen sind, welche die wechselseitige Verbindung der Stücke mit Hilfe von Montagemitteln wie beispielsweise Schrauben (6) gestatten, wobei bestimmte unter den Öffnungen in den zugordneten Endteilen der Frontotemporal-Stangen bzw. -Schienen und/oder der Halbbögen in Form von Langlöchern (12) vorliegen, welche eine Einstellung ihrer Überlappung und so eine Anpassung der Abmessungen des Extensor-Rings (Halo) gestatten.

3. Extensor-Ring (Halo) nach Anspruch 1, dadurch gekennzeichnet, daß die Frontotemporalstangen bzw. -schienen (1) und die Halbbögen durch Verbindungsstücke miteinander verbunden sind, welche die Enden der Frontotemporalstangen bzw.-schienen teilweise überlappen, daß in den gegenüberstehenden Teilen dieser Verbindungsstücke und der Frontotemporalstangen bzw. -schienen Öffnungen zu ihrer gegenseitigen starren Verbindung mit Hilfe von Montagemitteln wie beispielsweise Schrauben vorgesehen sind, wobei bestimmte unter den Öffnungen der Verbindungsstücke und/oder der Frontotemporalstangen bzw. -schienen Langlochform besitzen, um sie in einer Mehrzahl von Stellungen montieren zu können.

4. Extensor-Ring (Halo) nach Anspruch 3, dadurch gekennzeichnet, daß die Halbbögen (2) und die Frontotemporalstangen bzw.-schienen (1) durch ihre aneinder grenzenden Enden überlappende Platten miteinander verbunden sind, daß in den gegenüberstehend angeordneten Teilen der Halbbögen und der Verbindungsstücke Öffnungen für Montagemittel wie beispielsweise Schrauben vorgesehen sind, und daß bestimmte unter den Öffnungen dieser Stangen bzw. schienen und/oder dieser Stücke Langlochform besitzen, derart daß sie in einer Mehrzahl von Stellungen montiert werden können.

5. Extensor-Ring (Halo) nach einem der Ansprüche 1 bis 4, mit Occipital-Stangen bzw. -Schienen (3), die zur Anlage gegenüber dem Hinterhaupt des Patienten bestimmt sind, dadurch gekennzeichnet, daß die Occipitalstangen bzw.-schienen (3) mit den an die Frontotemporalstangen bzw. -schienen angrenzenden Enden (2a) der Halbbögen (2) oder der Verbindungsstücke mit Hilfe von Schrauben (6) oder dgl. verbunden sind, die in die Öffnungen (13, 14) der zu montierenden Stücke eingreifen, wobei wenigstens einige dieser Öffnungen die Form von Langlöchern (14) haben.

6. Extensor-Ring (Halo) nach einem der Ansprüche 1 bis 5, dadurch gekennzeichnet, daß die Frontotemporalstangen bzw. -schienen (1) und/oder die Occipitalstangen bzw.-schienen (3) Langlöcher (16) aufweisen, mit welchen Führungsstützen (19) von Stell-bzw. Zentrierschrauben (19a) oder dgl. in Eingriff stehen, die zur Fixierung des Extensor-Rings (Halos) auf der Schädelkalotte des Patienten bestimmt sind.

7. Extensor-Ring (Halo) nach Anspruch 6, dadurch gekennzeichnet, daß die Lauffläche der Führungsstützen (19) der Stell-bzw. Zentrierschrauben (19a) oder dgl. leicht sphärisch ist, um die Schrauben (19a) ausrichten zu können.

8. Extensor-Ring (Halo) nach einem der Ansprüche 1 bis 7, dadurch gekennzeichnet, daß die Halbbögen aus zwei in verstellbaren Lagen bzw. Stellungen montierbaren Teilen (31, 32) bestehen.

9. Extensor-Ring (Halo) nach Anspruch 8, dadurch gekennzeichnet, daß die Teile (31, 32) der Halbbögen mit Hilfe von Platten (33) montiert sind, welche Langlöcher (34) aufweisen, mit welchen Schrauben (35) oder dgl. in einstellbarer Lage in Eingriff stehen, die gleichzeitig mit Öffnungen (36,37) der Teile (31,32) in Eingriff stehen.

10. Extensor-Ring (Halo) nach einem der Ansprüche 1 bis 9, dadurch gekennzeichnet, daß das Ende (2a) eines der Halbbögen und/oder das Ende (1b) einer Frontotemporalstange bzw.-schiene (1) bezüglich einem ersten Teil (54) schwenkbar montiert ist, das seinerseits mit einem zweiten, in seiner Lage auf einer Stange (40) einer Brustkorb-Abstützung verstellbaren Teil (41) verbunden ist, wobei das genannte Ende beweglich mit einem Teil (50) verbunden ist, das an dem ersten Teil (54) gehaltert und mittels einer Schraube (46) oder dgl. translatorisch bezüglich diesem ersten Teil verstellbar ist.
